(19) **Europäisches Patentamt**
**European Patent Office**
**Office européen des brevets**

(11) **EP 2 039 674 B1**

(12) **EUROPEAN PATENT SPECIFICATION**

(45) Date of publication and mention
of the grant of the patent:
**27.06.2012 Bulletin 2012/26**

(51) Int Cl.:
**C07C 51/42** [(2006.01)] **C07C 57/04** [(2006.01)]
**C07C 51/36** [(2006.01)]

(21) Application number: **08164210.0**

(22) Date of filing: **12.09.2008**

(54) **Improved process for selective reduction of propionic acid from (meth)acrylic acid product streams**

Verbessertes Verfahren zur selektiven Reduktion von Propionsäure aus (Meth) acrylsäureproduktströmen

Procédé amélioré pour la réduction sélective d'acide propionique à partir de flux de produit d'acide méthacrylique

(84) Designated Contracting States:
**DE FR GB**

(30) Priority: **19.09.2007 US 994450 P**

(43) Date of publication of application:
**25.03.2009 Bulletin 2009/13**

(73) Proprietor: **Rohm and Haas Company**
**Philadelphia, PA 19106-2399 (US)**

(72) Inventors:
• **Han, Scott**
**Lawrenceville, NJ 08648 (US)**
• **Xu, Jinsuo**
**Ft. Washington, PA 19034 (US)**

(74) Representative: **Kent, Venetia Katherine**
**Patent Outsourcing Limited**
**1 King Street**
**Bakewell**
**Derbyshire DE45 1DZ (GB)**

(56) References cited:
**EP-A- 0 046 840        WO-A-03/045886**
**US-A- 5 959 143**

• **DATABASE WPI Week 200020 Thomson Scientific, London, GB; AN 2000-232942 XP002551890 & JP 2000 053611 A (MITSUBISHI CHEM CORP) 22 February 2000 (2000-02-22)**
• **DATABASE WPI Week 200048 Thomson Scientific, London, GB; AN 2000-527367 XP002551891 & JP 2000 169420 A (MITSUBISHI CHEM CORP) 20 June 2000 (2000-06-20)**
• **DATABASE CA [Online] CHEMICAL ABSTRACTS SERVICE, COLUMBUS, OHIO, US; SAKAI, YUKIO ET AL: "Manufacture of acrylic acid with inhibition of propionic acid formation" XP002551889 retrieved from STN Database accession no. 130:154077 & JP 11 035519 A (MITSUBISHI CHEMICAL INDUSTRIES LTD., JAPAN) 9 February 1999 (1999-02-09)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to an improved process for the selective reduction of propionic acid, hereinafter "PA", impurity from an acrylic acid, hereinafter "AA" stream.

(Meth)Acrylic acid (AA), one example of an unsaturated carboxylic acid, is used in a wide variety of applications. Typical end use applications include: acrylic plastic sheeting; molding resins; polyvinyl chloride modifiers; processing aids; acrylic lacquers; floor polishes; sealants; auto transmission fluids; crankcase oil modifiers; automotive coatings; ion exchange resins; cement modifiers; water treatment polymers; electronic adhesives; metal coatings; and acrylic fibers.

Propionic acid (PA), an impurity in the acrylic acid monomer, is an undesirable volatile organic compound which can affect product qualities of acrylic acid products. Thus, current commercial AA processes employing a two-step partial oxidation of propylene yield PA concentrations of less than 1,000 ppm, which is a typical specification level. However, AA made by the partial oxidation of propane, may contain between 3,000 and 30,000 ppm PA by weight. These concentrations of PA would pose significant product quality problems if they could not be removed from AA.

As used herein, the use of the term "(meth)" followed by another term such as acrylate refers to both acrylates and methacrylates. For example, the term "(meth)acrylate" refers to either acrylate or methacrylate; the term "(meth)acrylic" refers to either acrylic or methacrylic; the term "(meth)acrylic acid" refers to either acrylic acid or methacrylic acid.

The purification of PA from AA presents both a technical challenge and a potential economical burden on AA manufacture. AA and PA cannot be separated by conventional distillation due to their nearly identical boiling points. Furthermore, the extraction of PA from AA using common solvents, such as isopropyl acetate, toluene or diphenyl ether, is also unsuccessful due to their similarity in solubility.

Currently the only commercial technique available for effectively separating PA from AA is melt crystallization, as described in US 5504247. This technique, however, would require higher initial capital investment to lower the PA content down to the specification of less than 1000 ppm. Furthermore, the operation of a melt crystallizer is energy intensive. As propane oxidation becomes an economically attractive route to AA due to the rapid catalyst development in this field, low cost and efficient techniques for PA removal are needed.

A variety of processes involving a post stage reactor to selectively remove PA from AA stream have been disclosed. Unfortunately, the AA was substantially oxidized along with the removal of PA. For example, JP 2000053611 cited a process of lowering PA to 115ppm from 337ppm over catalyst containing MoFeCoO with AA yield loss up to 8.6%.

It is therefore an objective of the present invention to provide an improved catalyst for use in PA reduction from AA stream under oxidation conditions. As a result, it has unexpectedly been found that the most selective catalyst in PA reduction from AA stream is the same mixed metal oxide (MMO) that is used to prepare AA and PA from propane oxidation. Accordingly, it is an object of the invention to provide PA reduction catalyst for use in producing high purity AA monomers.

The present invention provides a process for selectively removing propionic acid from an acrylic acid stream comprising:

reacting an acrylic acid stream in the presence of a propionic acid reduction mixed metal oxide catalyst ;

wherein the mixed metal oxide catalyst comprises a mixed metal oxide comprising the empirical formula

$$A_a M_b N_c X_d Z_e O_f$$

wherein A is at least one element selected from the group consisting of Mo and W; M is at least one element selected from the group consisting of V and Ce; N is at least one element selected from the group consisting of Te, Sb and Se; X is at least one element selected from the group consisting of Nb, Ta, Ti, Al, Zr, Cr, Mn, Fe, Ru, Co, Rh, Ni, Pt, Sb, Bi, B, In, As, Ge, Sn, Li, Na, K, Rb, Cs, Fr, Be, Mg, Ca, Sr, Ba, Ra, Hf, Pb, P, Pm, Eu, Gd, Dy, Ho, Er, Tm, Yb and Lu; and Z is at least one element selected from the group consisting of Zn, Ga, Ir, Sm, Pd, Au, Ag, Cu, Sc, Y, Pr, Nd and Tb; and O is oxygen in oxide form and wherein, when $a = 1$, $b = 0.01$ to $1.0$, $c = 0.01$ to $1.0$, $d = 0.01$ to $1.0$, $e = 0$ to $0.1$, and f is dependent on the oxidation state of the other elements.

Preferably, the mixed metal oxides of the present invention have the formulae $Mo_a V_m Te_n Nb_x O_o$ and $W_a V_m Te_n Nb_x O_o$ wherein a, m, n, x and o are as previously defined.

The AA of the present invention may be produced by any convention technique known by those of ordinary skill in the art. Additionally, any conventional raw material feed may be used to produce AA so long as the AA product stream contains some amount of PA impurity. Specifically, the AA product stream contains greater than 1000, 500, or 100ppm of PA impurity. Examples of raw materials that can be used to produce AA in the present invention include, but are not limited to functionalized and multi-functionalized hydrocarbons such as aldehydes, alcohols, diols, etc., light alkanes and alkenes other than propane, such as propylene, biomass and other non-petroleum based sources of hydrocarbons. Specifically, the AA product stream can be the product stream of a propane or propylene oxidation process.

The AA product stream may be the product stream of either a single or multi-stage oxidation process.

The mixed metal oxide catalyst reacts with the formed AA to selectively reduce PA. The PA reduction mixed metal oxide catalyst may be installed in the same propane oxidation reactor with the propane oxidation catalyst, or in a separate PA reduction finishing reactor. This finishing step could be performed with or without separation of acid products before the finishing step. For example, in the case where propane is the main raw material in an oxidation reaction to produce AA, the stream out off the propane oxidation reactor may be directly fed to a separate PA reduction reactor without separation of acid products.

The reaction of the PA reduction mixed metal oxide catalyst and AA operates at a temperature of less than 325 °C with a residence time of 0.1 to 6 seconds. Alternatively, the operation or reaction temperature is less than 300 °C or 275 °C and the residence time is 0.1 to 3 seconds.

Conversely, the PA reduction catalyst can also be put in the same reactor with propane oxidation catalyst. It is preferred that PA reduction catalyst is loaded downstream of propane oxidation catalyst. It is also preferable that the reactor has different zone of temperature control so the PA reduction can be operated at a different temperature from the propane oxidation zone.

Additionally, oxygen may be injected into the PA reduction reactor if the oxygen concentration in the product stream from propane oxidation reactor is very low.

The PA reduction reaction may be operated in liquid phase other than vapor phase. Multiple AA streams may be combined together for PA reduction. This combination is advantageous because it lowers the operator/owner's capital investment. Furthermore, the PA reduction reaction may be combined with known separation methods such as distillation and melt crystallization to further purify the AA product to the desired grade specification.

PA reduction of the present invention can optionally take place as a part of an integrated AA production process containing a propylene generation step and downstream AA separation processes

The mixed metal oxides of the present invention may be prepared by processs commonly known to those of ordinary skill in the art. One non-limiting example of a process is disclosed herein.

In a first step, a mixture is formed by admixing metal compounds, preferably at least one of which contains oxygen, and at least one solvent in appropriate amounts to form the slurry or solution. Preferably, a solution is formed at this stage of the catalyst preparation. Generally, the metal compounds contain constituent elements A, M, N, O and X, as previously defined.

Suitable solvents include aqueous solutions and alcohols, including but not limited to, water, methanol, ethanol, propanol, and diols, etc., as well as other polar solvents known in the art. Typically, water is preferred. The water is any water suitable for use in chemical syntheses including, without limitation, distilled water and de-ionized water. The volume of water present is preferably a volume sufficient to keep the constituent elements substantially in solution long enough to avoid or minimize compositional and/or phase segregation during the preparation steps. Accordingly, the volume of water will vary according to the amounts and solubility of the materials combined. However, as stated above, the volume of water is preferably sufficient to ensure an aqueous solution is formed, at the time of mixing.

For example, when a mixed metal oxide of the formula $Mo_aV_bTe_cNb_xO_n$, is prepared, an aqueous solution of telluric acid, an aqueous solution of niobium oxalate and a mixture of ammonium paramolybdate may be sequentially added to an aqueous solution containing a predetermined amount of ammonium metavanadate, so that the atomic ratio of the respective metal elements would be in the prescribed proportions.

Once the mixture is formed, the water is removed by any suitable process, known in the art, to form a catalyst precursor. Such processs include, without limitation, vacuum drying, freeze drying, spray drying, rotary evaporation, and air drying. Vacuum drying is generally performed at pressures ranging from 1.3 kPa to 66.6 kPa. Freeze drying typically entails freezing the slurry or solution, using, for example, liquid nitrogen, and drying the frozen slurry or solution under vacuum. Spray drying is generally performed under an inert atmosphere such as nitrogen or argon, with an inlet temperature ranging from 125°C to 200°C and an outlet temperature ranging from 75°C to 150°C. Rotary evaporation is generally performed at a bath temperature of from 25°C to 90°C and at a pressure of from 1.3 kPa to 101.3 kPa, preferably at a bath temperature of from 40° to 90°C and at a pressure of from 1.3 kPa to 46.7 kPa, more preferably at a bath temperature of from 40°C to 60°C and at a pressure of from 1.3 kPa to 5.3 kPa. Air drying is performed at temperatures ranging from 25°C to 90°C. Rotary evaporation and air drying are typically preferred drying processs.

Once obtained, the mixed metal oxide catalyst precursor is calcined. The calcination may be conducted in an oxidizing atmosphere, but it is also possible to conduct the calcination in a non-oxidizing atmosphere, for example in an inert atmosphere or in vacuo. The inert atmosphere may be any material which is substantially inert, that is any material that does not react or interact with the mixed metal oxide catalyst precursor. Suitable examples include, without limitation, nitrogen, argon, xenon, helium or mixtures thereof. The inert atmosphere may or may not flow over the surface of the catalyst precursor. When the inert atmosphere does not flow over the surface of the catalyst, this is referred to as a static environment. When the inert atmosphere does flow over the surface of the mixed metal oxide catalyst precursor, the flow rate can vary over a wide range, for example at a space velocity of from 1 to 500 $hr^{-1}$.

The calcination is usually performed at a temperature of from 350°C to 850°C, preferably from 400°C to 700°C, more preferably from 500°C to 640°C. The calcination is performed for an amount of time suitable to form the aforementioned catalyst. Typically, the calcination is performed for from 0.5 to 30 hours, preferably from 1 to 25 hours, more preferably for from 1 to 15 hours, to obtain the desired mixed metal oxide catalyst.

In a preferred mode of operation, the mixed metal oxide catalyst precursor is calcined in two stages. In the first stage, the catalyst precursor is calcined in an oxidizing atmosphere (e.g., air) at a temperature of from 200°C to 400°C, preferably from 275°C to 325°C for from 15 minutes to 8 hours, preferably for 1 to 3 hours. In the second stage, the material from the first stage is calcined in a non-oxidizing environment (e.g., an inert atmosphere) at a temperature of from 500°C to 750°C, preferably for from 550°C to 650°C, for from 15 minutes to 8 hours, preferably for from 1 to 3 hours. Optionally, a reducing gas, such as, for example ammonia or hydrogen, may be added during the second stage calcination.

In one embodiment of the present invention, the catalyst precursor in the first stage is placed in the desired oxidizing atmosphere at room temperature and then raised to the first stage calcination temperature and held there for the desired first stage calcination time. The atmosphere is then replaced with the desired non-oxidizing atmosphere for the second stage calcination, the temperature is raised to the desired second stage calcination temperature and held there for the desired second stage calcination time.

Although any type of heating mechanism, e.g., a furnace, may be utilized during the calcination, it is preferred to conduct the calcination under a flow of the designated gaseous environment. Therefore, it is advantageous to conduct the calcination in a bed with continuous flow of the desired gas(es) through the bed of solid catalyst precursor particles.

With calcination, a catalyst is formed having the formula $A_aM_mN_nX_xO_o$ wherein A, M, N, X, O, a, m, n, x and o are as previously defined.

The starting materials for the above mixed metal oxide catalyst are not limited to those described above. A wide range of materials including, for example, oxides, nitrates, halides or oxyhalides, alkoxides, acetylacetonates and organometallic compounds may be used. For example, ammonium heptamolybdate may be utilized for the source of molybdenum in the catalyst. However, compounds such as $MoO_3$, $MoO_2$, $MoCl_5$, $MoOCl_4$, $Mo(OC_2H_5)_5$, molybdenum acetylacetonate, phosphomolybdic acid and silicomolybdic acid may also be utilized instead of ammonium heptamolybdate. Similarly, ammonium metavanadate may be utilized for the source of vanadium in the catalyst. However, compounds such as $V_2O_5$, $V_2O_3$, $VOCl_3$, $VCl_4$, $VO(OC_2H_5)_3$, vanadium acetylacetonate and vanadyl acetylacetonate may also be utilized instead of ammonium metavanadate. The tellurium source may include telluric acid, $TeCl_4$, $Te(OC_2H_5)_5$, $Te(OCH(CH_3)_2)_4$ and $TeO_2$. The niobium source may include ammonium niobium oxalate, $Nb_2O_5$, $NbCl_5$, niobic acid or $Nb(OC_2H_5)_5$ as well as the more conventional niobium oxalate.

A mixed metal oxide thus obtained, exhibits excellent catalytic activities. However, the same mixed metal oxide may be converted to a catalyst having improved catalytic performance by grinding.

Grinding may be performed by any conventional means known to those of ordinary skill in the art. Dry and wet grinding processs may be employed. In the case of dry grinding, a gas stream grinder may be used wherein coarse particles are permitted to collide with one another in a high speed gas stream. Additionally, grinding may be conducted not only mechanically but also by using a mortar or the like in the case of a small scale operation. In the case of wet grinding, grinding is conducted in a wet state by adding water or an organic solvent to the above mixed metal oxide. A conventional process of using a rotary cylinder-type medium mill or a medium-stirring type mill may be employed. The rotary cylinder-type medium mill is a wet mill of the type wherein a container for the object to be ground is rotated, and it includes, for example, a ball mill and a rod mill. The medium-stirring type mill is a wet mill of the type wherein the object to be ground, contained in a container is stirred by a stirring apparatus, and it includes, for example, a rotary screw type mill, and a rotary disc type mill.

The conditions for grinding may suitably be set to meet the nature of the above-mentioned mixed metal oxide, the viscosity, the concentration, etc. of the solvent used in the case of wet grinding, or the optimum conditions of the grinding apparatus. However, it is preferred that grinding is conducted until the average particle size of the ground catalyst precursor is no greater than 20$\mu$m, more preferably no greater than 5$\mu$m. As aforementioned, grinding improves the catalytic activities of the mixed metal oxide catalyst.

The catalytic activities of the mixed metal oxide catalyst may be further improved by adding a solvent to the ground catalyst precursor to form a solution or slurry, followed by drying again. There is no particular restriction as to the concentration of the solution or slurry, and it is common practice to adjust the solution or slurry so that the total amount of the starting material compounds for the ground catalyst precursor is from 10 to 60 wt %. The solution or slurry is then dried by a process such as spray drying, freeze drying, evaporation to dryness, or vacuum drying, preferably spray drying.

Additionally, the mixed metal oxide catalyst obtained may be impregnated with a variety of elements, including but not limited to Te and Nb, and re-calcined to further improve its performance.

Contacting the ground catalyst with certain organic or inorganic acids, such as for example oxalic acid in a methanol or water solution, at elevated temperature such as, 40°C -100°C also improves catalytic activity.

The mixed metal oxide catalyst obtained by the above-mentioned process may be used "as is" as a final catalyst, or it may be subjected to heat treatment at temperatures ranging from 200° to 700°C for a time period ranging from from 0.1 to 10 hours.

The mixed metal oxide catalyst thus obtained may be used by itself as a solid catalyst, but may be formed into a catalyst together with a suitable carrier such as silica, alumina, titania, aluminosilicate, diatomaceous earth or zirconia. Further, it may be molded into a suitable shape and particle size depending upon the scale or system of the reactor.

Alternatively, the metal components of the presently contemplated mixed metal oxide catalyst composition may be supported on materials such as for example, alumina, silica, silica-alumina, zirconia, and titania by conventional incipient wetness techniques. In one process, solutions containing the metals are contacted with the dry support such that the support is wetted; the resultant wetted material is dried, for example, at a temperature from 20°C to 200°C followed by calcination as described above. In another process, metal solutions are contacted with the support, typically in volume ratios of greater than 3:1, metal solution to support, and the solution is agitated such that the metal ions are ion-exchanged onto the support. The metal containing support is then dried and calcined as detailed above.

The present invention is described in more detail with reference to the following Example and Comparative Examples, which should not be construed as limiting the scope of the present invention.

EXAMPLES

PREPARATION OF THE CATALYST

Preparation of a mixed oxide catalyst represented by the formula:

$$Mo_1V_{0.285}Te_{0.21}Nb_{0.164}Pd_{0.01}O_x$$

where x is determined by the valences of the other metals in the MMO was prepared as follows:

1. Precalulated amounts of salts of Mo, V, and Te was dissolved in 200g DI water at 70°C in a two-liter round-bottom flask to give an orange solution.
2. Precalculated amounts of salts of Nb, Pd, and oxalic acid was dissolved in 180g DI water at room temperature in a 250 ml beaker
3. Concentrated nitric acid was added to the MoVTe solution at 70°C with stirring, the color deepened to red-orange.
4. The Nb solution was added to the MoVTe solution to give a orange-colored gel.
5. Water was removed to create a solid.
6. The solid was dried in a vacuum oven overnight at room temperature.
7. The orange solid was removed from the flask to yield a mixed metal oxide precursor.
8. The mixed metal oxide precursor was calcined in tube furnaces as follows: heated in air 10 °C/min to 275 °C, held at temperature for 30 minutes, switched to argon atmosphere, heated 2 °C/min to 615 °C, soaked 120 minutes.
9. The calcined solid material was broken and sieved through 10 mesh screen.
10. These particles were first stirred five hours in water at room temperature and then dried. Step 10 may be performed in combination with the following impregnation step, 11, without sacrificing catalyst performance.
11. The water-treated catalyst was then impregnated with an aqueous solution containing telluric acid and niobium ammonium oxalate. The water was removed.
12. The dried material was calcined to convert telluric acid and niobium ammonium oxalate to the corresponding oxides. The calcination was carried out first in air at 300°C for three hours, then in argon at 500°C for two hours.
13. The recalcined material was ground with a freezer/mill.
14. The ground material was extracted with oxalic acid in water at 100°C for 30min-5h. The solid material was recovered by filtration, and dried in a vacuum oven overnight.
15. The material was pressed and sieved to 14 - 20 mesh granules for reactor evaluation.

PREPARATION OF COMPARATIVE CATALYST 1: IRON PHOSPHATE ($FePO_4$)

Iron phosphate catalyst shows a unique selectivity for several oxidative dehydrogenation reactions, such as formation of methacrylic acid by oxidative dehydrogenation of isobutyric acid (Applied Catalysis A: General, 109, 135-146, 1994), and formation of pyruvic acid from lactic acid (Applied Catalysis A: General 234, 235-243, 2002). Iron phosphate was prepared according to literature procedure (Applied Catalysis A: General 234, 235-243, 2002), as shown below:

1 $Fe(OH)_3$ gel preparation: A 14%wt $NH_4OH$ solution (~90g) was added drop-wise to a solution containing 48.8g $Fe(NO_3)_{3.9}H_2O$ and 2000cc $H_2O$ under stirring, at room temperature.
2 Water was removed by decanting, then 16.6g 85% $H_3PO_4$ was added to the precipitate

3 The $H_3PO_4/Fe(OH)_3$ mixture was transferred to a flask, boiled for 3-5h to yield slightly brownish-white precipitate

4 The precipitate was filtered and washed with water to remove excess $H_3PO_4$

5 The filter cake was dried in an oven at 120°C overnight

6 The dried cake was ground and pressed into pellets

7 The pellets were calcined in flowing air at 400°C for 8 h at a ramp rate of 2°C/min and then the pellets were crushed to 14-20 mesh

PREPARATION OF COMPARATIVE CATALYST 2: $Cs_2Mo_{12}V_{1.5}P_2O_{45.8}$

The catalyst $Cs_2Mo_{12}V_{1.5}P_2O_{45.8}$ was prepared according to US patent 4370490, in which the catalyst $Cs_2Mo_{12}V_{1.5}P_2O_{45.8}$ showed good selectivity in the oxidative dehydrogenation of isobutyric acid to methacrylic acid. The following is a detailed procedure of the catalyst preparation:

1 Prepared solution "A": 2.88g 85% $H_3PO_4$ + 25 ml $H_2O$

2 Prepared solution "B": 26.8g 28% $NH_4OH$ + 48.3ml $H_2O$ + 26.5g $(NH_4)_6Mo_7O_{24}$ $4H_2O$

3 Added solution "A" to solution "B" under stirring to generate mixture "AB".

4 Prepared solution "C": 4.85g $CsNO_3$ + 50 ml $H_2O$

5 The mixture "AB" was added to solution "C" under stirring to generate a new mixture "ABC".

6 Prepared solution "D": 2.2g $NH_4VO_3$ + 35 ml 10% monoethanolamine in $H_2O$

7 The solution "D" was added to the mixture "ABC". The new mixture was called "ABCD".

8 10.21g diatomaceous earth and 2.05g Aerosil®200, obtained from DeGussa Chemicals, were added to the mixture "ABCD" as catalyst support.

9 The supported catalyst "ABCD/SiO$_2$" was dried over hot plate under stirring for 1h at 50°C and then dried using rotary evaporatoration under vacuum.

10 The dried "ABCD/SiO$_2$" was calcined in a box furnace at 110°C for 7h, then 300°C for 3h. The ramp rate was 5°C/min.

11 The calcined hard mass from step "10" was sieved to 14-20 mesh for testing. The final catalyst had a formula "$Cs_2Mo_{12}V_{1.5}P_2O_{45.8}/SiO_2$"

PREPARATION OF COMPARATIVE CATALYST 3: $Mo_{12}V_3W_{1.2}Cu_{1.2}Sb_{0.5}O_x$

The formula for catalyst composition 3 is $Mo_{12}V_3W_{1.2}Cu_{1.2}Sb_{0.5}O_x$, a material used in the process of converting acrolein to AA. The catalyst was prepared following the procedure described in US patent 5,959,143. The final catalyst was crushed to 14-20 mesh prior to testing.

1 Made solution "E". The following chemicals (a-e) were added in the following order to a 1000 cc rotary evaporation flask:

a) 300ml $H_2O$, heated to ~85°C

b) 7.67g ammonium metatungstate

c) 9.11g $NH_4VO_3$

d) 55g ammonium heptamolybdate

e) 1.89g $Sb_2O_3$

2 Made solution "F": 48ml H2O + 7.78g CuSO4.5H2O

3 Solution "F" was added to solution "E" dropwise over a period of 10-15 minutes period to form a slurry

4 The slurry mixture was dried in a rotary evaporation flask under vacuum at 50°C, and then vacuum dried overnight

5 The dried mixture was further dried at 120°C for 16h and calcined at 390°C for 5h. The ramp rate was 1°/min.

EXAMPLE 1

Each of catalyst of the present invention and comparative catalyst compositions was first evaluated in the oxidative dehydrogenation reaction of PA to see whether AA could be formed from PA oxidation. The test conditions were as follows: 4 mol% PA, 3mol% $O_2$, 33mol% $H_2O$, balance was $N_2$. The total reactant gas mixture flow rate was 80cc/min. The catalyst amount was ~5g (14-20 mesh). A once-through tubular reactor was filled with denstone, commercially available from Norton Chemicals, on both ends of the catalyst bed. The reactor temperature was 200-400°C. The products were analyzed by gas chromatography. The conversions listed in the table were generally calculated as follows:

$$PA\ Conv.\ (\%) = 100 \times [(\text{moles of PA in the feed} - \text{moles of PA in the product})/\text{moles of PA in the feed}]$$

$$AA\ Sel.\ (\%) = 100 \times [\text{moles of AA in the product}/(\text{moles of PA in the feed} - \text{moles of PA in the product})]$$

$$AA\ Yield\ (\%) = 100 \times (\text{moles of AA in the product}/\ \text{moles of PA in the feed})$$

The results are listed in Table 1

Table 1. Performance of the Various Oxidation Catalysts in PA Oxidative Dehydrogenation Reaction

| Catalyst | PA Conv. (%) | AA Sel. (%) | AA Yield (%) |
|---|---|---|---|
| COMPARATIVE EXAMPLE 1 | 27 | 2 | 0.5 |
| ($FePO_4$) | 60 | 2 | 1.2 |
| COMPARATIVE EXAMPLE 2 | 25 | 15 | 3.8 |
| ($Cs_2Mo_{12}V_{1.5}P_2O_{45.8}$) | 61 | 14 | 8.5 |
| COMPARATIVE EXAMPLE 3 | 26 | 6 | 1.6 |
| ($Mo_{12}V_3W_{1.2}Cu_{1.2}Sb_{0.5}O_x$) | 47 | 6 | 2.8 |
| CATALYST | 15 | 23 | 3.4 |
| ($Mo_1V_{0.285}Te_{0.21}Nb_{0.164}Pd_{0.01}O_x$) | 38 | 17 | 6.5 |
| | 69 | 12 | 8.3 |

All the catalysts tested above did show some selectivity to AA during PA oxidation. From Table 1, it can be seen that the $Mo_1V_{0.285}Te_{0.21}Nb_{0.164}Pd_{0.01}O_x$ catalyst gave the highest AA selectivities of 12-23% at comparable PA conversions to the other catalysts tested. These results show clearly the unique properties of the catalyst of the present invention relative to a range of other types of catalysts tested for PA conversion to AA.

EXAMPLE 2

Each catalyst was next evaluated using a mixed AA and PA feed with PA concentration around 4000ppm. The test results are reported in Table 2. The axis values in the table were calculated as follows:

$$AA\ loss\ (\%) = 100 \times [1 - (\text{moles of AA exited the reactor}/\ \text{moles of AA fed into the reactor})]$$

$$PA\ level\ in\ AA\ (ppm) = 1{,}000{,}000 \times (\text{moles of PA exited the reactor}/\ \text{moles of AA exited the reactor})$$

Table 2. Comparison of Efficacy of Multiple Catalysts in Selective Reduction of PA From an AA Stream

| Catalyst | Reactor T (°C) | PA Level in AA (ppm) | AA Loss (%) |
|---|---|---|---|
| COMPARATIVE EXAMPLE 1 (FePO$_4$) | 180 | 4062 | 0 |
| | 220 | 3858 | 2.5 |
| | 230 | 3694 | 3.5 |
| | 240 | 3425 | 4.4 |
| | 250 | 3223 | 5.4 |
| | 270 | 1870 | 23.2 |
| COMPARATIVE EXAMPLE 2 (Cs$_2$Mo$_{12}$V$_{1.5}$P$_2$O$_{45.8}$) | 180 | 4515 | 0 |
| | 230 | 4014 | 2.7 |
| | 270 | 4151 | 4.5 |
| | 290 | 4108 | 6.3 |
| | 310 | 4112 | 14.2 |
| | 330 | 3145 | 27.9 |
| COMPARATIVE EXAMPLE 3 (Mo$_{12}$V$_3$W$_{1.2}$Cu$_{1.2}$Sb$_{0.5}$O$_x$) | 180 | 3827 | 0 |
| | 230 | 3835 | 1.8 |
| | 250 | 3843 | 2 |
| | 270 | 3833 | 2.9 |
| | 290 | 3713 | 5.8 |
| | 310 | 3010 | 13.8 |
| | 320 | 2409 | 16.4 |
| | 330 | 1425 | 25.8 |
| | 340 | 536 | 34 |
| CATALYST (Mo$_1$V$_{0.285}$Te$_{0.21}$Nb$_{0.164}$Pd$_{0.01}$O$_x$) | 180 | 3987 | 0 |
| | 220 | 3824 | 2 |
| | 250 | 3565 | 2.3 |
| | 280 | 2583 | 3.7 |
| | 300 | 1350 | 5.9 |
| | 310 | 686 | 7.2 |
| | 320 | 257 | 8.6 |

As shown in Table 2, due to the much higher relative concentration of AA vs. PA (AA/PA ≈ 250), AA was inevitably consumed along with the oxidation of PA. However, the amount of AA consumed varied with respect to the different catalysts when PA was reduced. The mixed metal oxide catalyst (Mo$_1$V$_{0.285}$Te$_{0.21}$Nb$_{0.164}$Pd$_{0.01}$O$_x$) was by far the most selective catalyst. The catalyst of the present invention, as depicted in the catalyst of the present invention, exhibited only ~6% AA consumption when the PA level was reduced to approximately 1000 ppm, while the other catalyst compositions exhibited an AA consumption of at least 4 times greater to get PA concentration down to the same level. In particular, it is unexpected that the most effective catalyst used to remove PA was in fact the exact same catalyst used to initially form the PA in the propane oxidation reaction.

## Claims

1. A process for selectively removing propionic acid from an acrylic acid stream comprising:

   reacting an acrylic acid stream in the presence of a propionic acid reduction mixed metal oxide catalyst ;
   wherein the mixed metal oxide catalyst comprises a mixed metal oxide comprising the empirical formula

   AaMbNcXdZeOf

   wherein A is at least one element selected from the group consisting of Mo and W; M is at least one element selected from the group consisting of V and Ce; N is at least one element selected from the group consisting of Te, Sb and Se; X is at least one element selected from the group consisting of Nb, Ta, Ti, Al, Zr, Cr, Mn, Fe, Ru, Co, Rh, Ni, Pt, Sb, Bi, B, In, As, Ge, Sn, Li, Na, K, Rb, Cs, Fr, Be, Mg, Ca, Sr, Ba, Ra, Hf, Pb, P, Pm, Eu, Gd, Dy, Ho, Er, Tm, Yb and Lu; and Z is at least one element selected from the group consisting of Zn, Ga, Ir, Sm, Pd, Au, Ag, Cu, Sc, Y, Pr, Nd and Tb; and O is oxygen in oxide form and wherein, when a = 1, b = 0.01 to 1.0, c = 0.01 to 1.0, d = 0.01 to 1.0, e = 0 to 0.1, and f is dependent on the oxidation state of the other elements.

2. The process of claim 1 wherein the catalyst is at least one oxide of a metal selected from the group comprising Mo, V, Te, Nb and O or combinations thereof.

3. The process of claim 1 wherein the catalyst is at least one oxide of a metal selected from the group comprising W, V, Te, Nb, and O or combinations thereof.

4. The process of claim 1 wherein the acrylic acid stream has greater than 100 ppm of propionic acid as impurity.

5. The process of claim 1 wherein the acrylic acid stream is the product stream of a propane or propylene oxidation process.

6. The process of claim 1 wherein the acrylic acid stream is the product stream of a single or multi-stage oxidation process.

7. The process of claim 1 wherein the propionic acid reduction mixed metal oxide catalyst is in the same reactor as the reactor used to produce the acrylic acid stream.

8. The process of claim 1 wherein the propionic acid reduction mixed metal oxide catalyst is in a separate reactor from the reactor used to produce the acrylic acid stream.

9. The process of claim 1 wherein the propionic acid reduction step is part of an integrated acrylic acid production process wherein the integrated acrylic acid production process comprises a propylene generation step and downstream acrylic acid separation processes.

10. The process of claim 1 wherein the propionic acid reduction step is combined with distillation or melt crystallization.

**Patentansprüche**

1. Verfahren zum selektiven Entfernen von Propionsäure aus einem Acrylsäurestrom, umfassend:

das Umsetzen eines Acrylsäurestroms in der Gegenwart eines Propionsäurereduktions-Mischmetalloxidkatalysators,
wobei der Mischmetalloxidkatalysator ein Mischmetalloxid umfasst, welches die empirische Formel

$$A_a M_b N_c X_d Z_e O_f$$

umfasst, wobei A mindestens ein Element, ausgewählt aus der Gruppe, bestehend aus Mo und W ist, M mindestens ein Element, ausgewählt aus der Gruppe, bestehend aus V und Ce ist, N mindestens ein Element, ausgewählt aus der Gruppe, bestehend aus Te, Sb und Se ist, X mindestens ein Element, ausgewählt aus der Gruppe, bestehend aus Nb, Ta, Ti, Al, Zr, Cr, Mn, Fe, Ru, Co, Rh, Ni, Pt, Sb, Bi, B, In, As, Ge, Sn, Li, Na, K, Rb, Cs, Fr, Be, Mg, Ca, Sr, Ba, Ra, Hf, Pb, P, Pm, Eu, Gd, Dy, Ho, Er, Tm, Yb und Lu ist, und Z mindestens ein Element, ausgewählt aus der Gruppe, bestehend aus Zn, Ga, Ir, Sm, Pd, Au, Ag, Cu, Sc, Y, Pr, Nd und Tb ist, und O Sauerstoff in Oxidform ist, und wobei, wenn a = 1, b = 0,01 bis 1,0, c = 0,01 bis 1,0, d = 0,01 bis 1,0, e = 0 bis 0,1, und f abhängig von der Oxidationsstufe der anderen Elemente ist.

2. Verfahren nach Anspruch 1, wobei der Katalysator mindestens ein Oxid eines Metalls, ausgewählt aus der Gruppe, umfassend Mo, V, Te, Nb und O oder Kombinationen davon, ist.

3. Verfahren nach Anspruch 1, wobei der Katalysator mindestens ein Oxid eines Metalls, ausgewählt aus der Gruppe, bestehend aus W, V, Te, Nb und O oder Kombinationen davon, ist.

**4.** Verfahren nach Anspruch 1, wobei der Acrylsäurestrom mehr als 100 ppm Propionsäure als Verunreinigung aufweist.

**5.** Verfahren nach Anspruch 1, wobei der Acrylsäurestrom der Produktsstrom eines Propan- oder Propylenoxidationsverfahrens ist.

**6.** Verfahren nach Anspruch 1, wobei der Acrylsäurestrom der Produktstrom eines ein- oder mehrstufigen Oxidationsverfahrens ist.

**7.** Verfahren nach Anspruch 1, wobei der Propionsäurereduktions-Mischmetalloxidkatalysator sich in dem gleichen Reaktor befindet wie der Reaktor, welcher zur Herstellung des Acrylsäurestroms verwendet wird.

**8.** Verfahren nach Anspruch 1, wobei der Propionsäurereduktions-Mischmetalloxidkatalysator sich in einem von dem zur Herstellung des Acrylsäurestroms verwendeten Reaktor getrennten Reaktor befindet.

**9.** Verfahren nach Anspruch 1, wobei der Propionsäurereduktionsschritt Teil eines integrierten Acrylsäureherstellungsverfahrens ist, wobei das integrierte Acrylsäureherstellungsverfahren einen Propylenerzeugungsschritt und Downstream-Acrylsäuretrennverfahren umfasst.

**10.** Verfahren nach Anspruch 1, wobei der Propionsäurereduktionsschritt mit Destillation oder Schmelzkristallisation kombiniert wird.


## Revendications

**1.** Procédé d'élimination sélective de l'acide propionique d'un courant d'acide acrylique comprenant :

la réaction d'un courant d'acide acrylique en présence d'un catalyseur d'oxyde de métaux mixtes de réduction d'acide propionique ;
le catalyseur d'oxyde de métaux mixtes comprenant un oxyde de métaux mixtes possédant la formule empirique :

$$A_a M_b N_c X_d Z_e O_f$$

dans laquelle A est au moins un élément choisi dans le groupe consistant en Mo et W ; M est au moins un élément choisi dans le groupe consistant en V et Ce ; N est au moins un élément choisi dans le groupe consistant en Te, Sb et Se ; X est au moins un élément choisi dans le groupe consistant en Nb, Ta, Ti, Al, Zr, Cr, Mn, Fe, Ru, Co, Rh, Ni, Pt, Sb, Bi, B, In, As, Ge, Sn, Li, Na, K, Rb, Cs, Fr, Be, Mg, Ca, Sr, Ba, Ra, Hf, Pb, P, Pm, Eu, Gd, Dy, Ho, Er, Tm, Yb et Lu ; et Z est au moins un élément choisi dans le groupe consistant en Zn, Ga, Ir, Sm, Pd, Au, Ag, Cu, Sc, Y, Pr, Nd et Tb ; et 0 est un atome d'oxygène sous forme d'oxyde et dans laquelle lorsque a = 1, b = 0,01 à 1,0, c = 0,01 à 1,0, d = 0,01 à 1,0, e = 0 à 0,1, et f dépend de l'état d'oxydation des autres éléments.

**2.** Procédé selon la revendication 1, dans lequel le catalyseur est au moins un oxyde d'un métal choisi dans le groupe comprenant Mo, V, Te, Nb et 0 ou des combinaisons de ceux-ci.

**3.** Procédé selon la revendication 1, dans lequel le catalyseur est au moins un oxyde d'un métal choisi dans le groupe comprenant W, V, Te, Nb et 0 ou des combinaisons de ceux-ci.

**4.** Procédé selon la revendication 1, dans lequel le courant d'acide acrylique comprend plus de 100 ppm d'acide propionique en tant qu'impureté.

**5.** Procédé selon la revendication 1, dans lequel le courant d'acide acrylique est le courant du produit d'un procédé d'oxydation de propane ou de propylène.

**6.** Procédé selon la revendication 1, dans lequel le courant d'acide acrylique est le courant du produit d'un procédé d'oxydation en une ou plusieurs étapes.

**7.** Procédé selon la revendication 1, dans lequel le catalyseur d'oxyde de métaux mixtes de réduction d'acide propionique est dans le réacteur utilisé pour produire le courant d'acide acrylique.

**8.** Procédé selon la revendication 1, dans lequel le catalyseur d'oxyde de métaux mixtes de réduction d'acide propionique est dans un réacteur distinct du réacteur utilisé pour produire le courant d'acide acrylique.

**9.** Procédé selon la revendication 1, dans lequel l'étape de réduction de l'acide propionique fait partie d'un procédé de production d'acide acrylique intégré, le procédé de production d'acide acrylique intégré comprenant une étape de préparation de propylène et des procédés de séparation de l'acide acrylique en aval.

**10.** Procédé selon la revendication 1, dans lequel l'étape de réduction de l'acide propionique est combinée à une distillation ou une cristallisation de la masse fondue.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5504247 A
- JP 2000053611 B
- US 4370490 A
- US 5959143 A

**Non-patent literature cited in the description**

- *Applied Catalysis A: General,* 1994, vol. 109, 135-146
- *Applied Catalysis A: General,* 2002, vol. 234, 235-243